# FASCICULE DE BREVET EUROPEEN

(11) **EP 0 737 512 B1**
(45) Date de publication et mention de la délivrance du brevet: **05.01.2000**
(21) Numéro de dépôt: 96400679.5
(22) Date de dépôt: 29.03.1996
(51) Int. Cl.: B01J 20/08, C07C 7/12, C08F 6/02

(54) **Procédé pour l'adsorption de composés organométalliques chélatés**
Verfahren zur Adsorption von organometallischen Chelatverbindungen
Process for adsorption of chelated organometallic compounds

(30) Priorité: 10.04.1995 FR 9504259
(43) Date de publication de la demande: 16.10.1996
(73) Titulaire: INSTITUT FRANCAIS DU PETROLE, 92852 Rueil-Malmaison Cedex (FR)
(72) Inventeur: Taxil, Bernard, 30340 Salindres (FR); Nedez, Chistophe, 92600 Asnières sur Seine (FR)

(56) Documents cités:
- EP-A- 0 067 708
- EP-A- 0 379 394
- WO-A-90/04007
- DE-A- 3 029 802
- FR-A- 1 383 076
- FR-E- 73 525

## Description

L'invention concerne un procédé pour l'adsorption de composés organométalliques chélatés dans lequel on met en contact lesdits composés avec des billes à base d'alumine de caractéristiques adaptées. Elle concerne plus particulièrement un procédé pour la purification des polyoléfines obtenues par polymérisation d'oléfines en présence de catalyseurs de coordination. Elle concerne également des billes d'alumine comprenant un composé organométallique chélaté.

Les polyoléfines sont préparées généralement par polymérisation de monomères avec ajout possible de comonomères tels que le 1-butène, le 1-octène,... en présence de catalyseurs de polymérisation comprenant des éléments des groupes IVB, VB, VIB du classement périodique des éléments et plus particulièrement le vanadium, le titane, le zirconium. Ces catalyseurs comprennent également comme agents réducteurs des composés organométalliques (alkyl métallique), hydrures métalliques ou hydroxydes de métaux. Ces catalyseurs, généralement appelés catalyseurs de transition, présentent une grande activité catalytique pour la polymérisation d'oléfines.

Cependant, une fois la polymérisation achevée, les polyoléfines obtenues se trouvent polluées par les résidus métalliques provenant des catalyseurs et il est donc indispensable de les épurer avant leur utilisation, pour éviter toute toxicité comme une coloration ou une dégradation parasites.

En outre, les procédés de polymérisation des oléfines comprennent généralement une étape de récupération des monomères n'ayant pas réagi au cours de la polymérisation et des solvants contenus dans les polyoléfines, ces solvants et monomères étant recyclés dans l'unité de polymérisation. La présence des métaux dans ces composés engendre des problèmes de corrosion de l'installation.

Pour éliminer les résidus métalliques issus des catalyseurs, un procédé consiste à mettre en contact le milieu issu de la polymérisation avec des composés organiques. De ce fait, il se crée une réaction de complexation entre les résidus métalliques et les composés organiques introduits, conduisant à des composés organométalliques chélatés.

Ensuite, afin de séparer ces composés organométalliques chélatés des polyoléfines, il est connu d'utiliser différents adsorbants, notamment des alumines.

Parmi les adsorbants utilisés, il est avantageux d'utiliser des adsorbants sous forme de billes qui peuvent être manipulées plus aisément que de la poudre ou des blocs de formes variées. En effet, un produit sous forme de billes peut être véhiculé tant pour le chargement que le déchargement des colonnes d'épuration par des systèmes pneumatiques par exemple.

Parmi les adsorbants, l'alumine peut être aisément mise sous forme de billes.

Un but de la présente invention est de proposer des billes d'alumine pour l'adsorption des composés organométalliques chélatés présentant un taux d'adsorption amélioré par rapport à ceux des produits de l'art antérieur.

Dans ce but, l'invention concerne un procédé pour l'adsorption de composés organométalliques chélatés dans lequel on met en contact lesdits composés avec des billes à base d'alumine issues d'une mise en forme par agglomération d'une poudre d'alumine par technologie tournante et présentant un volume de pores de diamètre supérieur à 100 Å d'au moins 0,10 cm³/g, un volume de pores de diamètre supérieur à 1 µm d'au moins 0,05 cm³/g et un diamètre d'au plus 4 mm.

L'invention concerne en outre des billes d'alumine comprenant un composé organométallique chélaté obtenues après la mise en contact selon le procédé défini ci-dessus.

L'invention concerne tout d'abord, un procédé pour l'adsorption de composés organométalliques chélatés dans lequel on met en contact lesdits composés avec des billes à base d'alumine issues d'une mise en forme par agglomération d'une poudre d'alumine par technologie tournante et présentant un volume de pores de diamètre supérieur à 100 Å d'au moins 0,10 cm³/g, un volume de pores de diamètre supérieur à 1 µm d'au moins 0,05 cm³/g et un diamètre d'au plus 4 mm.

De manière préférentielle, le procédé met en oeuvre des billes présentant un volume de pores de diamètre supérieur à 100 Å supérieur à 0,15 cm³/g et un volume de pores de diamètre supérieur à 1 µm supérieur à 0,08 cm³/g.

De manière encore plus préférentielle, les billes présentent un volume de pores de diamètre supérieur à 100 Å supérieur à 0,2 cm³/g et un volume de pores de diamètre supérieur à 1 µm supérieur à 0,1 cm³/g.

Le volume des pores de diamètre supérieur à 100 Å, respectivement à 1 µm, représente le volume cumulé créé par tous les pores de taille supérieure à un diamètre de 100 Å, respectivement à 1 µm. Ces volumes sont mesurés par la technique de la pénétration du mercure, dans laquelle on applique la loi de Kelvin.

Selon le procédé de l'invention, les billes peuvent avantageusement présenter un diamètre inférieur à 3 mm, de préférence inférieur à 2,5 mm. Les diamètres des billes sont mesurés à l'aide d'un pied à coulisse.

Les procédés de préparation des billes utilisables dans le procédé selon l'invention sont connus de l'homme du métier.

Par exemple un procédé de préparation de ces billes, présentant une porosité et un diamètre contrôlés, peut consister à former des billes d'alumine par agglomération d'une poudre d'alumine. Suite à cette agglomération, les billes obtenues peuvent être soumises à différentes opérations destinées à améliorer leur résistance mécanique telles qu'un mûrissement par maintien dans une atmosphère à taux d'humidité contrôlée, suivi d'une calcination puis d'une imprégnation des billes par une solution d'un ou plusieurs acides et un traitement hydrothermal en atmosphère confinée. Enfin, les billes sont séchées et calcinées de manière à être activées. Par exemple, les billes peuvent être calcinées à une température située entre 300 et 1000 °C, de préférence entre 300 et 800 °C.

Ce type de procédé permet d'obtenir des billes de dimensions et de répartitions de pores contrôlées, ces dimensions et ces répartitions étant, en général, créées pendant l'étape d'agglomération.

La porosité peut être créée par différentes moyens comme le choix de la granulométrie de la poudre d'alumine ou l'agglomération de plusieurs poudres d'alumine de différentes granulométries. Une autre méthode consiste à mélanger à la poudre d'alumine, avant ou pendant l'étape d'agglomération, un composé, appelé porogène, disparaissant totalement par chauffage et créant ainsi une porosité dans les billes.

Comme composés porogènes utilisés, on peut citer, à titre d'exemple, la farine de bois, le charbon de bois, le soufre, des goudrons, des matières plastiques ou émulsions de matières plastiques telles que le polychlorure de vinyle, des alcools polyvinyliques, la naphtaline ou analogues. La quantité de composés porogènes ajoutés n'est pas critique et est déterminée par le volume poreux désiré.

La poudre d'alumine utilisée comme matière de départ peut être obtenue par des procédés classiques tels que le procédé par précipitation ou gel, et le procédé par déshydratation rapide d'un hydroxyde d'alumine tel que l'hydrate de Bayer (hydrargillite). Cette dernière alumine est celle préférée de l'invention.

L'agglomération des billes selon l'invention s'effectue directement sur la poudre d'alumine par technologie tournante. On entend par technologie tournante tout appareil dans lequel l'agglomération s'effectue par mise en contact et rotation du produit à granuler sur lui-même. Comme appareil de ce type, on peut citer le drageoir tournant, le tambour tournant.

Le contrôle des volumes des pores de diamètre donné peut également être réalisé au cours de cette étape d'agglomération par un réglage adéquat du débit d'introduction de la poudre d'alumine et éventuellement d'eau, de la vitesse de rotation de l'appareil ou lors de l'introduction d'une amorce de mise en forme.

Avantageusement, les billes mises en oeuvre dans le procédé selon l'invention présentent une surface spécifique d'au moins 100 m²/g, de préférence supérieure à 150 m²/g, encore plus préférentiellement supérieure à 200 m²/g. Cette surface spécifique est une surface BET. On entend par surface BET, la surface spécifique déterminée par adsorption d'azote conformément à la norme ASTM D 3663-78 établie à partir de la méthode BRUNAUER - EMMETT - TELLER décrite dans le périodique "The Journal of the american Society", 60, 309 (1938).

Le procédé selon l'invention permet d'obtenir des taux d'adsorption des composés organométalliques chélatés améliorés, qui peuvent atteindre plus de 60%, ce dernier taux représentant le taux de métal adsorbé par les billes par rapport à la quantité de départ de métal introduite dans le milieu réactionnel et dans les conditions définies dans le test d'adsorption ci-après.

D'une manière préférentielle, les billes peuvent comprendre au moins un composé d'un élément choisi dans le groupe comprenant les alcalins, les alcalino-terreux. Ce composé de l'élément peut être un oxyde, un hydroxyde, un sel de l'élément ou un mélange de ceux-ci. On peut citer, à titre d'exemple, en plus des hydroxydes, les sulfates, nitrates, halogénures, acétates, formiates, carbonates et plus généralement les sels d'acides carboxyliques par exemple.

On utilise de préférence les éléments choisis parmi le sodium, le potassium et le calcium.

La teneur du composé de l'élément choisi dans le groupe des alcalins, des alcalino-terreux peut être comprise entre 15 mmole et 150 mmole pour 100 g d'alumine, de préférence entre 15 et 100 mmole pour 100 g d'alumine.

L'incorporation de ces éléments peut être effectuée selon l'enseignement de la demande de brevet EP-A-0 379 394.

La présente invention concerne plus particulièrement le procédé mettant en oeuvre les billes décrites ci-dessus pour l'adsorption de tout composé organométallique chélaté, plus particulièrement à base des métaux choisis parmi ceux des groupes IVB, VB, VIB, VIIB, VIII, IB, IIB et encore plus particulièrement ceux à base de vanadium, de titane, de zirconium ou de cuivre.

Le procédé selon l'invention convient spécialement pour l'adsorption de tout composé organométallique qui a été chélaté par des composé organiques tels que l'acétylacétone, le 2-éthylhexanediol-1,3, le di-2-éthyl-hexylphosphate.

De ce fait, le procédé selon l'invention peut convenir pour la purification des polyoléfines obtenues par polymérisation d'oléfines en présence d'un système de catalyseur de coordination. Le procédé de purification peut être du type de celui qui a été décrit plus haut dans l'introduction de la présente description et dans lequel les billes sont mises en contact avec le milieu issu de la polymérisation préalablement mis en présence de composés organiques.

Des billes d'alumine comprenant un composé organométallique chélaté, lesdites billes sont obtenues après la mise en contact selon le procédé de l'invention des composés organométalliques avec les billes d'alumine issues d'une mise en forme par agglomération d'une poudre d'alumine par technologie tournante. Ces billes présentent un volume de pores de diamètre supérieur à 100 Å d'au moins 0,10 cm³/g, un volume de pores de diamètre supérieur à 1 µm d'au moins 0,05 cm³/g et un diamètre d'au plus 4 mm.

Dans le procédé selon l'invention, lesdites billes d'alumine sont mises en contact avec les composés organométalliques et les adsorbent. A l'issue du procédé d'adsorption des composés organométalliques, les billes sont retirées du réacteur et on obtient des billes d'alumine issues d'une mise en forme par agglomération d'une poudre d'alumine par technologie tournante, présentant un volume de pores de diamètre supérieur à 100 Å d'au moins 0,10 cm³/g, un volume de pores de diamètre supérieur à 1 µm d'au moins 0,05 cm³/g et un diamètre d'au plus 4 mm sur lesquelles sont adsorbées les composés organométalliques.

Ces billes peuvent être directement utilisées en tant que catalyseurs métalliques supportés dans tout type de catalyse par métaux précieux adapté à la nature du métal adsorbé.

Les exemples suivants illustrent l'invention, sans en limiter, toutefois, sa portée.

### EXEMPLES

### Test d'adsorption

Les tests d'adsorption ont été conduits sur des billes d'alumine activées au préalable à 300°C durant 2 h afin d'éliminer toute trace d'humidité suite à leur stockage et afin de pouvoir comparer leur efficacité dans des conditions identiques.

Les billes sont introduites dans un bêcher contenant du vanadium chélaté par l'acétylacétonate (VO(acac)₂) présent dans 200 ml de toluène à la concentration de 0,1% (en poids par rapport au volume de toluène). Elles sont laissées, sous agitation, au contact du composé durant 48 h à 25°C à l'abri de l'air. Le taux d'adsorption du vanadium chélaté par l'acétylacétonate par l'alumine est contrôlé par évolution de la concentration de la solution mesurée par spectroscopie UV-visible.

Toutes les billes testées dans les exemples ont été mises en forme à partir d'alumine hydrargillite à l'aide d'un drageoir tournant.

### Exemple 1 : influence des volumes des pores de diamètre supérieur à 100 Å et 1 µm.

| | Diamètre (mm) | Surface spécifique (m²/g) | V100 Å (cm³/g) | V1 µm (cm³/g) | Taux d'adsorption |
|---|---|---|---|---|---|
| Billes 1 comparatives | 2,2 | 324 | 0,06 | 0,04 | 55,4% |
| Billes 2 | 1,9 | 344 | 0,17 | 0,09 | 64% |
| Billes 3 | 1,9 | 333 | 0,26 | 0,15 | 71,5% |
| Billes 4 | 1,9 | 350 | 0,27 | 0,20 | 81,2% |
| Billes 5 | 1,9 | 308 | 0,30 | 0,23 | 78,6% |

On constate que le taux d'adsorption des billes 1, présentant un volume de pores de diamètre supérieur à 100 Å de 0,06 cm³/g et un volume de pores de diamètre supérieur à 1 µm de 0,04 cm³/g est de 55,4 %, tandis que le taux d'adsorption des billes 2 à 5, qui vérifient les caractéristiques des billes selon l'invention, est supérieur à 64%.

### Exemple 2 : influence du diamètre des billes.

| | Diamètre (mm) | Surface spécifique (m²/g) | V100 Å (cm³/g) | V1 µm (cm³/g) | Taux d'adsorption |
|---|---|---|---|---|---|
| Billes 6 | 1,2 | 175 | 0,70 | 0,34 | 85,4% |
| Billes 7 | 1,7 | 175 | 0,70 | 0,34 | 84,1% |
| Billes 8 | 2,4 | 175 | 0,70 | 0,34 | 76,3% |
| Billes 9 | 3,4 | 175 | 0,70 | 0,34 | 69,5% |
| Billes 10 | 3,15 | 335 | 0,26 | 0,15 | 60,7% |
| Billes 11 comparatives | 4,5 | 335 | 0,26 | 0,15 | 53,6% |
| Billes 12 comparatives | 4,70 | 335 | 0,26 | 0,15 | 52,2% |
| Billes 13 comparatives | 5,5 | 335 | 0,26 | 0,15 | 46,4% |

On constate que le taux d'adsorption des billes 11 à 13, présentant un diamètre supérieur à 4 mm, ne dépasse pas 60%, contrairement aux billes 6 à 10 qui vérifient les caractéristiques des billes selon l'invention.

## Revendications

1. Procédé pour l'adsorption de composés organométalliques chélatés caractérisé en ce qu'on met en contact lesdits composés avec des billes à base d'alumine issues d'une mise en forme par agglomération d'une poudre d'alumine par technologie tournante et présentant un volume de pores de diamètre supérieur à 100 Å d'au moins 0,10 cm³/g, un volume de pores de diamètre supérieur à 1 µm d'au moins 0,05 cm³/g et un diamètre d'au plus 4 mm.

2. Procédé selon la revendication 1 caractérisé en ce que la surface spécifque des billes est d'au moins 100 m²/g.

3. Procédé selon l'une des revendications 1 ou 2 caractérisé en ce que les billes comprennent au moins un composé d'un élément choisi dans le groupe comprenant les alcalins, les alcalino-terreux.

4. Procédé selon la revendication 3 caractérisé en ce que la teneur du composé de l'élément choisi dans le groupe des alcalins, des alcalino-terreux est comprise entre 15 mmole et 150 mmole pour 100 g d'alumine.

5. Procédé selon l'une des revendications 1 à 4 caractérisé en ce que les billes sont mises en forme par agglomération d'une poudre d'alumine par drageoir ou tambour tournant.

6. Procédé selon l'une des revendications 1 à 5 pour l'adsorption des composés organométalliques chélatés à base des métaux choisis parmi ceux des groupes IVB, VB, VIB, VIIB, VIII, IB, IIB.

7. Procédé selon l'une des revendications 1 à 6 pour l'adsorption des composés organométalliques chélatés à base de vanadium, de titane, de zirconium ou de cuivre.

8. Procédé selon l'une des revendications 1 à 7 pour l'adsorption des composés organométalliques chélatés à base des chélates choisis parmi l'acétylacétone, le 2-éthylhexanediol-1,3, le di-2-éthyl-hexylphosphate.

9. Procédé selon l'une des revendications 1 à 8 pour la purification des polyoléfines obtenues par polymérisation d'oléfines en présence d'un système de catalyseur de coordination.

## Claims

1. Process for the adsorption of cheated organometallic compounds, characterized in that the said compounds are placed in contact with alumina-based beads produced by forming by agglomeration of an alumina powder using a rotational technique and exhibiting a volume of pores of diameter greater than 100 Å of at least 0.10 cm³/g, a volume of pores of diameter greater than 1 µm of at least 0.05 cm³/g and a diameter of not more than 4 mm.

2. Process according to claim 1, characterized in that the specific surface of the beads is at least 100 m²/g.

3. Process according to either of claims 1 and 2, characterized in that the beads include at least one compound of an element chosen from the group including the alkali and alkaline-earth metals.

4. Process according to claim 3, characterized in that the content of the compound of the element chosen from the group of the alkali and alkaline-earth metals is between 15 mmol and 150 mmol per 100 g of alumina.

5. Process according to one of claims 1 to 4, characterized in that the beads are formed by agglomeration of an alumina powder using a coating pan or rotary drum.

6. Process according to one of claims 1 to 5 for the adsorption of cheated organometallic compounds based on metals chosen from those of the groups IVB, VB, VIB, VIIB, VIII, IB and IIB.

7. Process according to one of claims 1 to 6 for the adsorption of cheated organometallic compounds based on vanadium, titanium, zirconium or copper.

8. Process according to one of claims 1 to 7 for the adsorption of cheated organometallic compounds based on chelates chosen from acetylacetone, 2-ethyl-1,3-hexanediol and di-2-ethylhexyl phosphate.

9. Process according to one of claims 1 to 8 for the purification of the polyolefins obtained by polymerization of olefins in the presence of a coordination catalyst system.

## Patentansprüche

1. Verfahren für die Adsorption von chelatgebundenen Organometallverbindungen,
dadurch gekennzeichnet, dass
die besagten Verbindungen in Kontakt mit Kugeln auf Aluminiumbasis gebracht werden, die durch die Verdichtung eines Aluminiumpulvers im Drehverfahren geformt werden und ein Volumen von mindestens 0,10 cm³/g an Poren mit einem Durchmesser von über 100 Å, ein Volumen von mindestens 0,05 cm³/g an Poren mit einem Durchmesser von über 1 µm sowie ein Durchmesser von maximal 4 mm aufweisen.

2. Verfahren nach Anspruch 1 dadurch gekennzeichnet, dass die Oberflächenkennzahl der Kugeln mindestens 100 m²/g beträgt.

3. Verfahren nach einem der Ansprüche 1 oder 2 dadurch gekennzeichnet, dass die Kugeln mindestens eine Verbindung aus einem Element enthalten, das aus der Gruppe ausgesucht wird, die die Alkali und die Erdalkali umfaßt.

4. Verfahren nach Anspruch 3 dadurch gekennzeichnet, dass der Gehalt der Verbindung aus dem in der Gruppe der Alkali und Erdalkali ausgesuchten Element zwischen 15 mMol und 150 mMol pro 100 g Aluminium beträgt.

5. Verfahren nach einem der Ansprüche 1 bis 4 dadurch gekennzeichnet, dass die Kugeln durch Verdichtung eines Aluminiumpulvers in einer Apparatur zur Kornbildung von Katalysatoren oder in der Drehtrommel geformt werden.

6. Verfahren nach einem der Ansprüche 1 bis 5 für die Adsorption der chelatgebundenen Qrganometallverbindungen auf der Grundlage der Metalle, die unter denen aus der Gruppe IVB, VB, VIB, VIIB, IB, IIB ausgesucht wurden.

7. Verfahren nach einem der Ansprüche 1 bis 6 für die Adsorption der chelatgebundenen Organometallverbindungen auf der Grundlage von Vanadium, Titan, Zirkonium oder Kupfer.

8. Verfahren nach einem der Ansprüche 1 bis 7 für die Adsorption der chelatgebundenen Organometallverbindungen auf der Grundlage der Chelate, die unter Acetylaceton, 2-Ethylhexandiol-1,3 und dem di-2-Ethylhexylphosphat ausgesucht wurden.

9. Verfahren nach einen der Ansprüche 1 bis 8 für die. Reinigung der Polyolefine, die durch Olefinpolymerisation in Gegenwart von einem Koordinationskatalysatorsystem erzeugt wurden.
